# EUROPEAN PATENT APPLICATION

(11) **EP 1 862 547 A1**
(43) Date of publication of application: **05.12.2007**
(21) Application number: 06729890.1
(22) Date of filing: 24.03.2006
(51) Int. Cl.: C12N 15/11, C12Q 1/68, A61K 38/00, C12Q 1/04

(54) **METHOD OF DETECTING MYCOPLASMA**

(30) Priority: 25.03.2005 JP 2005089572
(71) Applicant: CHUGAI SEIYAKU KABUSHIKI KAISHA, Tokyo, 115-8543 (JP)
(72) Inventor: IWAKIRI, Shouji, c/o Chugai Seiyaku K.K., Tokyo 115-8543 (JP)
(74) Representative: Harding, Charles Thomas
(86) International application number: PCT/JP2006/305942
(87) International publication number: WO 2006/104031

(57) **Abstract**

An object of the present invention is to provide a primer capable of detecting a mycoplasma A. laidlawii (Acholeplasma laidlawii) sensitively and specifically. In order to achieve this object, the primer of the present invention is a primer for detecting A. laidlawii and for amplifying at least either one of polynucleotides containing the following nucleotide sequences (A) and (B) or partial sequences thereof: (A) a nucleotide sequence from position 589 to position 987 of SEQ ID NO: 1 or a sequence complementary thereto; (B) a nucleotide sequence from position 604 to position 975 of SEQ ID NO: 1 or a sequence complementary thereto. First, the region (A) is amplified and then the region (B) is amplified for the amplified product (A). Thus, A. laidlawii can be detected more sensitively and specifically.

## Description

### Technical Field

The present invention relates to a primer, a primer set, a kit for detecting a mycoplasma and a mycoplasma detection method, and a method for producing a pharmaceutical composition using the same.

### Background Art

A mycoplasma is a prokaryotic organism that lacks a cell wall. It is known that in the case where a cultured cell is contaminated with a mycoplasma, intrinsic functions or characteristics of the cell are affected in various ways. It is concerned that the production of a pharmaceutical product using such a contaminated cell may lead to a serious situation. As major mycoplasmas that are detected in cultured cells, the following 6 species can be exemplified.
Mycoplasma hyorhinis (hereinafter referred to as M. hyorhinis)
Mycoplasma orale (hereinafter referred to as M. orale)
Mycoplasma arginini (hereinafter referred to as M. arginini)
Mycoplasma fermentans (hereinafter referred to as M. fermentans)
Mycoplasma hominis (hereinafter referred to as M. hominis)
Acholeplasma laidlawii (hereinafter referred to as A. laidlawii)
Therefore, in a pharmaceutical production process, it is necessary to confirm that a cultured cell is not contaminated with a mycoplasma. In order to confirm this, a method capable of detecting all of the above-mentioned 6 species of mycoplasmas is necessary.

As a guideline for detecting a mycoplasma, the Japanese Pharmacopoeia, Fourteenth Edition (2001) is used and primers for detecting a mycoplasma are described therein (Non-patent document 1, SEQ ID NOS: 6 to 9). However, these primers have a disadvantage that their detection sensitivity to A. laidlawii among the above-mentioned 6 species of mycoplasmas is low. Further, a primer for detecting A. laidlawii has also been disclosed in a document (see, for example, Patent document 1), however, it has a disadvantage that it amplifies DNA of B. subtilis or the like.

Therefore, a primer capable of detecting A. laidlawii sensitively and also as specifically as possible has been demanded.
Patent document 1: JP-A-2004-305207
Non-patent document 1: The Japanese Pharmacopoeia, Fourteenth Edition (2001), General Information, "9. Mycoplasma Testing for Cell Substrates used for the Production of Biotechnological/Biological Products" pp. 1238-1241

### Disclosure of the invention

### Problems that the Invention is to Solve

Accordingly, an object of the present invention is to provide a primer capable of detecting A. laidlawii sensitively and specifically.

### Means for Solving the Problems

In order to achieve the above object, a primer of the present invention is a primer for detecting A. laidlawii and for amplifying at least either one of polynucleotides containing the following nucleotide sequences (A) and (B) or partial sequences thereof:
(A) a nucleotide sequence from position 589 to position 987 of SEQ ID NO: 1; and
(B) a nucleotide sequence from position 604 to position 975 of SEQ ID NO: 1.

### Advantage of the Invention

By using the primer of the present invention, A. laidlawii can be detected sensitively and specifically. The primer of the present invention can also be used in, for example, a nested PCR, and therefore, it becomes possible to detect A. laidlawii more sensitively and specifically. By using the primer of the present invention and conventional primers capable of detecting the above-mentioned 5 species of mycoplasmas in combination, it becomes possible to detect the above-mentioned 6 species of mycoplasmas at a time with a sufficient sensitivity to the respective mycoplasmas.

Further, with the use of the primer of the present invention, it becomes possible to provide a primer set for detecting A. laidlawii, a kit for detecting A. laidlawii, a method for detecting A. laidlawii or a mycoplasma, and a method for producing a pharmaceutical composition including the step of examining a mycoplasma.

### Brief Description of the Drawings

[Fig. 1] Figs. 1A and 1B are electrophoresis photographs showing an example of results of detecting a mycoplasma in a CSF-producing cell culture solution.
[Fig. 2] Figs. 2A and 2B are electrophoresis photographs showing an example of results of detecting a mycoplasma in an anti-IL-6 receptor antibody-producing cell culture solution.

### Best Mode for Carrying Out the Invention

Examples of the primer of the present invention for amplifying a polynucleotide containing the nucleotide sequence (A) or a partial sequence thereof include a primer capable of hybridizing to at least either one of polynucleotides consisting of the following nucleotide sequences (U1) and (D1):
(U1) a sequence complementary to a nucleotide sequence from position 571 to position 588 of SEQ ID NO: 1; and
(D1) a nucleotide sequence from position 988 to position 1004 of SEQ ID NO: 1, and the like.
Hereinafter, the primer capable of hybridizing to the polynucleotide consisting of the nucleotide sequence (U1) is referred to as a forward primer U1 of the present invention, and the primer capable of hybridizing to the polynucleotide consisting of the nucleotide sequence (D1) is referred to as a reverse primer D1 of the present invention.

Examples of the forward primer U1 of the present invention include a primer composed of a nucleotide sequence of SEQ ID NO: 2 (hereinafter also referred to as AF1-3), a primer composed of a nucleotide sequence in which one or more nucleotides have been substituted, added or deleted in the nucleotide sequence of SEQ ID NO: 2, a primer composed of a nucleotide sequence with an identity of 60% or more, preferably 70% or more, more preferably 88% or more to the nucleotide sequence of SEQ ID NO: 2, and the like.

Examples of the reverse primer D1 of the present invention include a primer composed of a nucleotide sequence of SEQ ID NO: 4 (hereinafter also referred to as AR1-3), a primer composed of a nucleotide sequence in which one or more nucleotides have been substituted, added or deleted in the nucleotide sequence of SEQ ID NO: 4, a primer composed of a nucleotide sequence with an identity of 60% or more, preferably 70% or more, more preferably 88% or more to the nucleotide sequence of SEQ ID NO: 4, and the like.

Examples of the primer of the present invention for amplifying a polynucleotide containing the nucleotide sequence (B) or a partial sequence thereof include a primer capable of hybridizing to at least either one of polynucleotides consisting of the following nucleotide sequences (U2) and (D2):
(U2) a sequence complementary to a nucleotide sequence from position 587 to position 603 of SEQ ID NO: 1; and
(D2) a nucleotide sequence from position 976 to position 995 of SEQ ID NO: 1, and the like.
Hereinafter, the primer capable of hybridizing to the polynucleotide consisting of the nucleotide sequence (U2) is referred to as a forward primer U2 of the present invention, and the primer capable of hybridizing to the polynucleotide consisting of the nucleotide sequence (D2) is referred to as a reverse primer D2 of the present invention.

Examples of the forward primer U2 of the present invention include a primer composed of a nucleotide sequence of SEQ ID NO: 3 (hereinafter also referred to as AF2-3), a primer composed of a nucleotide sequence in which one or more nucleotides have been substituted, added or deleted in the nucleotide sequence of SEQ ID NO: 3, a primer composed of a nucleotide sequence with an identity of 60% or more, preferably 70% or more, more preferably 88% or more to the nucleotide sequence of SEQ ID NO: 3, and the like.

Examples of the reverse primer D2 of the present invention include a primer composed of a nucleotide sequence of SEQ ID NO: 5 (hereinafter also referred to as AR2-3), a primer composed of a nucleotide sequence in which one or more nucleotides have been substituted, added or deleted in the nucleotide sequence of SEQ ID NO: 5, a primer composed of a nucleotide sequence with an identity of 60% or more, preferably 70% or more, more preferably 88% or more to the nucleotide sequence of SEQ ID NO: 5, and the like.

The primer set of the present invention is a primer set for detecting a mycoplasma A. laidlawii including 2 or more types of primers, and includes at least 2 types of primers of the present invention.

It is preferred that the primer set of the present invention can be used in a nested PCR method. In this case, the primer set of the present invention preferably includes as a primer set for a primary PCR, for example, the forward primer U1 and the reverse primer D1 of the present invention, and as a primer set for a secondary PCR, for example, the forward primer U2 and the reverse primer D2 of the present invention.

The detection kit of the present invention is a mycoplasma detection kit for detecting a mycoplasma by gene amplification, and includes the primer of the present invention or the primer set of the present invention as a primer or a primer set to be used for the gene amplification for detecting a mycoplasma A. laidlawii.

As another embodiment, the detection kit of the present invention further includes one or more primers to be used for the gene amplification for detecting at least one mycoplasma selected from the group consisting of Mycoplasma hyorhinis, Mycoplasma orale, Mycoplasma arginini, Mycoplasma fermentans and Mycoplasma hominis. The detection kit of the present invention preferably includes, for example, at least one primer selected from the group consisting of a primer composed of a nucleotide sequence of SEQ ID NO: 6 (hereinafter also referred to as F1), a primer composed of a nucleotide sequence of SEQ ID NO: 7 (hereinafter also referred to as F2), a primer composed of a nucleotide sequence of SEQ ID NO: 8 (hereinafter also referred to as R1) and a primer composed of a nucleotide sequence of SEQ ID NO: 9 (hereinafter also referred to as R2) as the primer to be used for the gene amplification for detecting at least one mycoplasma selected from the group consisting of M. hyorhinis, M. orale, M. arginini, M. fermentans and M. hominis.

The detection method of the present invention is a detection method for detecting A. laidlawii, including amplifying at least either one of polynucleotides containing the following nucleotide sequences (A) and (B) or partial sequences thereof and detecting the amplified polynucleotide:
(A) a nucleotide sequence from position 589 to position 987 of SEQ ID NO: 1 or a sequence complementary thereto; and
(B) a nucleotide sequence from position 604 to position 975 of SEQ ID NO: 1 or a sequence complementary thereto.
   In the detection method of the present invention, it is preferred that the primer of the present invention, the primer set of the present invention or the kit of the present invention is used in the amplification of the polynucleotides containing the nucleotide sequences (A) and (B) or partial sequences thereof.

The production method of the present invention is a method for producing a pharmaceutical composition, and includes the step of examining a mycoplasma in a produced pharmaceutical composition, wherein the examination step is carried out by the detection method of the present invention.

In the case where the pharmaceutical composition contains a polypeptide, it is preferred that the production method of the present invention includes, for example, the steps of:
(i) culturing a host cell containing a gene encoding the polypeptide contained in the pharmaceutical composition;
(ii) collecting a polypeptide expressed in the host cell in (i);
(iii) obtaining a sample for examination from the polypeptide collected in (ii); and
(iv) examining that a mycoplasma is not present in the sample obtained in (iii) by the detection method of the present invention.

### Mycoplasma

Mycoplasmas are bacteria classified in the class Mollicutes, and include bacteria belonging to the genus Mycoplasma in the family Mycoplasmataceae, the genus Ureaplasma in the family Mycoplasmataceae, the genus Acholeplasma in the Acholeplasmataceae, the genus Spiroplasma in the Spiroplasmataceae and the like. With the use of the primer of the present invention, A. laidlawii can be detected sensitively and specifically. Further, with the use of one of the primer sets of the present invention, it is possible to detect, for example, 6 species of mycoplasmas, M. hyorhinis, M. orale, M. arginini, M. fermentans, M. hominis and A. laidlawii with the same sensitivity to the respective 6 species of mycoplasmas. Incidentally, the sequences of the above-mentioned 6 species of mycoplasmas have been already known, and the DNA databank accession numbers (GenBank Accession ID) of these 16SrRNA gene sequences are M24658, M24659, U15794, M24289, AJ002269 and M23932 (SEQ ID NO: 1), respectively.

### Primer

In the case where A. laidlawii is detected by a gene amplification method using the primer of the present invention, a polynucleotide to be amplified is, for example, a polynucleotide containing (A) a nucleotide sequence from position 589 to position 987 of SEQ ID NO: 1 or a sequence complementary thereto, (B) a nucleotide sequence from position 604 to position 975 of SEQ ID NO: 1 or a sequence complementary thereto, or a partial sequence thereof. Further, in the case where the amplification of the polynucleotide containing the nucleotide sequence (A) is carried out using the primers U1 and D1 (see the above description) of the present invention, the resulting amplified product becomes, for example, a polynucleotide containing a nucleotide sequence from position 571 to position 1004 of SEQ ID NO: 1 or a sequence complementary thereto if the length of the primers are included. In the case where the amplification of the polynucleotide containing the nucleotide sequence (B) is carried out using the primers U2 and D2 (see the above description) of the present invention, the resulting amplified product becomes, for example, a polynucleotide containing a nucleotide sequence from position 587 to position 995 of SEQ ID NO: 1 or a sequence complementary thereto if the length of the primers are included. Incidentally, the nucleotide sequence of SEQ ID NO: 1 is a nucleotide sequence (M23932) of 16SrRNA gene of A. laidlawii. The gene amplification method is not particularly limited as described below, and for example, a conventionally known nucleic acid amplification method can be employed, however, preferably it is a PCR method.

Examples of the forward primer of the present invention include primers capable of hybridizing to a polynucleotide consisting of a sequence complementary to a nucleotide sequence from position 535 to position 603 of SEQ ID NO: 1, preferably from position 548 to position 603 of SEQ ID NO: 1, more preferably from position 558 to position 603 of SEQ ID NO: 1. Among these, the above-mentioned forward primers U1 and U2 are particularly preferred. Further, examples of the reverse primer of the present invention include primers capable of hybridizing to a polynucleotide consisting of a nucleotide sequence from position 976 to position 1019 of SEQ ID NO: 1, preferably from position 976 to position 1012 of SEQ ID NO: 1. Among these, the above-mentioned reverse primers D1 and D2 are particularly preferred. It is not necessary that these primers hybridize to the entire region of the above-mentioned polynucleotide, and it is only necessary that these primers hybridize to a partial region thereof.

A condition for the hybridization can be appropriately selected by a person skilled in the art. Examples of the condition for the hybridization include a low stringent condition. The low stringent condition is, for example, a condition of 42°C with 5 x SSC and 0.1% SDS, more preferably a condition of 50°C with 2 x SSC and 0.1% SDS in the washing after hybridization. More preferred examples of the condition for hybridization include a high stringent condition. The high stringent condition is, for example, a condition of 65°C with 0.1 x SSC and 0. 1% SDS. However, a plurality of factors such as a temperature and a salt concentration are considered as factors that have an influence on the stringency of hybridization, and by appropriately selecting these factors by a person skilled in the art, a similar stringency can be achieved. Incidentally, in the present invention, annealing is also included in the hybridization.

The forward primer of the present invention is preferably the forward primers U1 and U2, more preferably the primer AF1-3 composed of a nucleotide sequence of SEQ ID NO: 2 and the primer AF2-3 composed of a nucleotide sequence of SEQ ID NO: 3. Further, the reverse primer of the present invention is preferably the reverse primers D1 and D2, more preferably the primer AR1-3 composed of a nucleotide sequence of SEQ ID NO: 4 and the primer AR2-3 composed of a nucleotide sequence of SEQ ID NO: 5.

The primer of the present invention may also be a primer composed of a nucleotide sequence in which one or more nucleotides have been substituted, added or deleted in the nucleotide sequence of any of the above-mentioned primers AF1-3, AF2-3, AR1-3 and AR2-3, or may also be a primer composed of a nucleotide sequence with an identity of 60% or more, preferably 70% or more, more preferably 88% or more to the nucleotide sequence of any of the above-mentioned primers AF1-3, AF2-3, AR1-3 and AR2-3. A condition for the annealing is not particularly limited and can be appropriately selected by a person skilled in the art. However, as a specific condition, for example, a condition described in Examples described below can be employed.

The number of nucleotides to be substituted, added or deleted is not particularly limited and varies depending on the length of the primer or the like. However, it is, for example, 1 to 5, preferably 1 to 3, more preferably 1 to 2, further more preferably 1. Here, nucleotide insertion is included in the nucleotide addition.

The above-mentioned identity of a nucleotide sequence can be determined by using the algorithm BLAST by Karlin and Altschul (Proc. Natl. Acad. Sci. USA, 90: 5873-5877, 1993). The programs called BLASTN and BLASTX have been developed based on this algorithm (Altschul et al., J. Mol. Biol., 215: 403-410, 1990). In the case where a nucleotide sequence is analyzed by BLASTN based on BLAST, the parameters can be set, for example, score = 100 and wordlength = 12. The specific techniques of these analysis methods are known (http://www.ncbi.nlm. nih.gov.). The above-mentioned identity in the primer of the present invention is 60% or more, preferably 70% or more, more preferably 88% or more, further more preferably 94% or more.

The primer of the present invention is not particularly limited as long as it has a nucleotide sequence that satisfies the above-mentioned requirement, and may be any primer such as a DNA primer, an RNA primer, or a primer using a DNA or RNA analog. Further, a main strand that constitutes the primer is not particularly limited to a strand formed by a phosphodiester bond, and may be, for example, a phosphothioate strand having not phosphorus (P), but sulfur (S) as a backbone or a strand composed of a peptide nucleic acid linked with a peptide bond. Also, the nucleotide may be a nucleotide capable of forming a complementary base pair. In nature, the primer is composed of 5 types of bases, adenine, guanine, cytosine, thymine and uracil, however, it may also be an analog such as bromodeoxyuridine.

The length of the primer is generally 7-mer to 100-mer, preferably 15-mer to 40-mer, more preferably 18-mer to 30-mer. The hybridization of the primer of the present invention to a template DNA can be carried out under a condition known to a person skilled in the art, and for example, a condition described in Examples described below can be employed.

The primer of the present invention can be produced by a method known in this technical field. For example, a phosphotriethyl method, a phosphodiester method, or an automated method thereof is employed, or a method using an automated synthesizer can be exemplified.

### Primer set

The primer set in the present invention refers to a set of 2 or more types of primers. For example, in a PCR method, in the case where a specific region is amplified, two primers (a forward primer and a reverse primer) designed to sandwich the region to be amplified are necessary. Therefore, the primer set of the present invention preferably includes 2 or more types of primers of the present invention, more preferably includes a set of the forward primer of the present invention and the reverse primer of the present invention capable of amplifying a polynucleotide containing the nucleotide sequence (A) and/or (B) or a partial sequence thereof. Further, the primer set of the present invention may be a primer set including primers for amplifying different regions simultaneously or a primer set including primers to be used in a nested PCR.

The nested PCR is a method in which a gene is amplified by carrying out PCR by using a first primer set, and then, an inner region of the amplified gene (a PCR product) is amplified by using a second primer set. The second primer set is designed to be located at a position inner than that of the first primer set, however, both of the primers may be located at an inner side, or only one of the primers may be located at an inner side. The nested PCR is an effective method capable of suppressing a non-specific amplification.

The primer set of the present invention is preferably a primer set capable of detecting A. laidlawii using a nested PCR. In this case, it is preferred that the polynucleotide containing the nucleotide sequence (A) or a partial sequence thereof is amplified by a primary PCR and the polynucleotide containing the nucleotide sequence (B) or a partial sequence thereof is amplified by a secondary PCR. Therefore, it is preferred that the primer set of the present invention includes as a forward primer for the primary PCR, for example, the forward primer U1 preferably AF1-3, as a reverse primer for the primary PCR, for example, the reverse primer D1 preferably AR1-3, as a forward primer for the secondary PCR, for example, the forward primer U2, preferably AF2-3, and as a reverse primer for the secondary PCR, for example, the reverse primer D2, preferably AR2-3.

Further, in addition to the primers of the present invention for detecting A. laidlawii, the primer set of the present invention may include primers capable of detecting 5 species of mycoplasmas, M. hyorhinis, M. orale, M. arginini, M. fermentans and M. hominis. Examples of the primers capable of detecting the above-mentioned 5 species of mycoplasmas include primers F1 F2, R1 and R2 (SEQ ID NOS: 6, 7, 8, and 9) for a nested PCR described in the Japanese Pharmacopoeia, Fourteenth Edition (2001).

### Kit

The detection kit of the present invention is a mycoplasma detection kit for detecting a mycoplasma by gene amplification and includes the primer of the present invention for detecting a mycoplasma A. laidlawii. The species of mycoplasma other than A. laidlawii to be detected by the detection kit of the present invention is not particularly limited, however, for example, at least one species of the above-mentioned 5 species of mycoplasmas, M. hyorhinis, M. orale, M. arginini, M. fermentans and M. hominis can be exemplified. In this case, it is preferred that the above-mentioned primers F1, F2, R1 and R2 are included in the detection kit of the present invention. A member to be included in the detection kit of the present invention other than the above-mentioned primers is not particularly limited, however, examples thereof include various reagents such as enzymes (such as polymerase), buffers, and dNTPs.

### Detection method

The method for detecting a mycoplasma A. laidlawii of the present invention is a method in which at least either one of the polynucleotides containing the nucleotide sequences (A) and (B) or partial sequences thereof is amplified by a gene amplification method and the resulting amplified polynucleotide is detected, and is preferably carried out using, for example, the primer, primer set or detection kit of the present invention. The method for amplifying the polynucleotide is not particularly limited, and a conventionally known method can be used, however, for example, a PCR method, a nested PCR method or the like can be used. The PCR method is a technique well known to a person skilled in the art. Further, in order to improve the detection accuracy, it is preferred to use a nested PCR method. A condition for the PCR can be appropriately determined by a person skilled in the art, however, for example, the PCR can be carried out using a condition described in Examples of the present invention. A method for detecting an amplified product by PCR is not particularly limited, and examples thereof include electrophoresis using an agarose gel or the like, a method using a DNA chip and the like.

As a method for simultaneously detecting 6 species of mycoplasmas, M. hyorhinis, M. orale, M. arginini, M. fermentans, M. hominis and A. laidlawii by the nested PCR method, for example, a method in which a primary PCR is carried out for a test sample using U1 (preferably AF1-3) and F1 as the forward primers and D1 (preferably AR1-3) and R1 as the reverse primers, and a secondary PCR is carried out for the resulting amplified product using U2 (preferably AF2-3) and F2 as the forward primers and D2 (preferably AR2-3) and R2 as the reverse primers can be exemplified.

### Pharmaceutical composition

In the present invention, the pharmaceutical composition refers to a composition produced for the use of administering a human aiming at treatment, prevention or the like. A method for producing the pharmaceutical composition of the present invention is a production method including the step of examining a mycoplasma in a sample for examination of the pharmaceutical composition, and the examination step is carried out using the primer, primer set, detection kit or detection method of the present invention. The step of examining a mycoplasma is important in the production of any pharmaceutical composition, however, it is particularly important in the case where the step of culturing a cell is included in a production process for the pharmaceutical composition.

### Sample for examination

The sample for examination in the present invention generally refers to a sample taken out from a produced pharmaceutical composition, and is preferably a sample obtained by taking out a portion from a composition derived from the above-mentioned cultured cell or the like. The amount of the sample is not particularly limited, and may be an amount with which the detection of a mycoplasma can be carried out.

### Polypeptide contained in pharmaceutical composition

An active ingredient contained in the pharmaceutical composition of the present invention may be any substance such as a low molecular weight compound, a polypeptide or a nucleotide, however, it is preferably a polypeptide. In the present invention, the polypeptide is not particularly limited and may be any polypeptide. As the polypeptide, any protein including a biologically active substance such as erythropoietin or a granulocyte colony-stimulating factor, an antibody, a receptor and the like can be exemplified. A gene encoding such a polypeptide is generally incorporated in a vector and the vector is transformed into a host.

The vector is not particularly limited as long as in the case where E. coli is used as a host, for example, it has an "ori" to be amplified in E. coli and also has a selection gene, for example, a gene resistant to a drug such as ampicillin, tetracycline, kanamycin, or chloramphenicol. Examples of such a vector include M13-series vectors, pUC-series vectors, pBR322, pBluescript, pCR-Script and the like. Further, in the case where it is intended to perform subcloning and excision of cDNA, as the vector, for example, pGEM-T, pDIRECT, pT7 and the like can be exemplified. Further, as the E. coli being a host, for example, JM109, DH5α, HB101, XL1Blue and the like can be exemplified. In the case where it is intended to produce the above-mentioned polypeptide, it is preferred that the vector has a promoter that can achieve efficient expression in E. coli being a host. Examples of the promoter include a lacZ promoter (Ward et al., Nature (1989) 341, 544-546; FASEB J. (1992) 6, 2422-2427), an araB promoter (Better et al., Science (1988) 240, 1041-1043), a T7 promoter and the like. Further, examples of the expression vector include pGEX-5X-1 (manufactured by Pharmacia), a "QIAexpress system" vector (manufactured by QIAGEN), pEGFP, pET and the like. Incidentally, in the case where the above-mentioned pET is used, as a host E. coli, BL21 in which T7 RNA polymerase is expressed is preferred. Further, in the vector, a signal sequence for polypeptide secretion may be contained. In the case where the periplasm of E. coli is allowed to produce a polypeptide, a pelB signal sequence (Lei, S. P. et al., J. Bacteriol. (1987) 169, 4379) may be used as the signal sequence for polypeptide secretion. The introduction of the vector into a host cell can be carried out using, for example, a calcium chloride method or an electroporation method.

Other than the above-mentioned vectors in the case of using E. coli as a host, examples of the vector include expression vectors derived from mammals such as pcDNA3 (manufactured by Invitrogen), pEGF-BOS (Nucleic Acids. Res. 1990, 18 (17), p5322), pEF, and pCDM8; expression vectors derived from insect cells such as a "Bac-to-BAC baculovairus expression system" vector (manufactured by GIBCO BRL) and pBacPAK8; expression vectors derived from plants such as pMH1 and pMH2; expression vectors derived from animal viruses such as pHSV, pMV, and pAdexLcw; expression vectors derived from retroviruses such as pZIPneo; expression vectors derived from yeast such as a "Pichia Expression Kit" vector (manufactured by Invitrogen), pNV11 and SP-Q01; expression vectors derived from Bacillus subtilis such as pPL608 and pKTH50; and the like.

In the case where it is intended to express the polypeptide in an animal cell, such as a CHO cell, a COS cell, or an NIH3T3 cell, it is preferred that the vector has a promoter necessary for the expression thereof in such a cell, for example, an SV40 promoter (Mulligan et al., Nature (1979) 277, 108), an MMLV-LTR promoter, an EF1α promoter (Mizushima et al., Nucleic Acids Res. (1990) 18, 5322), a CMV promoter or the like. It is more preferred that the vector has a gene for selecting a transformed cell such as a gene resistant to a drug such as neomycin or G418. Examples of the vector having such a characteristic include pMAM, pDR2, pBK-RSV, pBK-CMV, pOPRSV, pOP13 and the like. Further, in the case where it is intended to stably express a gene and to amplify the gene copy number in a cell, a method in which a vector (such as pCHOI) having a DHFR gene that compensates for the deficiency is introduced into a CHO cell deficient in nucleic acid synthetic pathways and the vector is amplified with methotrexate (MTX) can be exemplified. Further, in the case where it is intended to transiently express a gene, a method in which a COS cell having a gene expressing SV40 T antigen on the chromosome is used and transformation is carried out with a vector (such as pcD) having the SV40 replication origin can be exemplified. As the replication origin, those derived from polyoma virus, adenovirus, bovine papilomavirus (BPV) and the like can also be used. Further, in order to amplify the gene copy number in a host cell system, the expression vector can include as a selection marker, an aminoglycoside transferase (APH) gene, a thymidine kinase (TK) gene, an E. coli xanthine guanine phosphoribosyl transferase (Ecogpt) gene, a dihydrofolate reductase (dhfr) gene or the like.

The host into which the vector is introduced is not particularly limited, and for example, E. coli, various animal cells and the like can be used as described above. As a production system for producing the polypeptide, there are in vitro and in vivo production systems. Examples of the in vitro production system include a production system using a eukaryotic cell and a production system using a prokaryotic cell. In the case where the eukaryotic cell is used, for example, an animal cell, a plant cell or a fungal cell can be used as the host. As the animal cell, mammalian cells such as CHO (J. Exp. Med. (1995) 108, 945), COS, 3T3, myeloma, BHK (baby hamster kidney), HeLa and Vero cells; amphibian cells such as Xenopus laevis oocytes (Valle, et al., Nature (1981) 291, 358-340); and insect cells such as Sf9, Sf21 and Tn5 are known. As the CHO cell, in particular, dhfr-CHO ( Proc. Natl. Acad. Sci. USA (1980) 77, 4216-4220) or CHO K-1 (Proc. Natl. Acad. Sci. USA (1968) 60, 1275), both of which are a CHO cell deficient in the DHFR gene, can be preferably used. Among the animal cells, in the case of aiming at mass expression, the CHO cell is particularly preferred. The introduction of the vector into the host cell can be carried out by, for example, a calcium phosphate method, a DEAE-dextran method, a method using cationic ribosome DOTAP (manufactured by Boehringer Mannheim), an electroporation method, lipofection or the like. As the plant cell, for example, a cell derived from Nicotiana tabacum is known as a polypeptide production system and may be subjected to callus culture. As the fungal cell, for example, yeast, Saccharomyces cerevisiae belonging to the genus Saccharomyces, filamentous bacteria, Aspergillus niger belonging to the genus Aspergillus and the like are known. In the case where the prokaryotic cell is used, there is a production system using a bacterial cell. Examples of the bacterial cell include E. coli such as JM109, DH5α and HB101, and other than these, Bacillus subtilis is known. Such a cell is transformed by a desired DNA, and the resulting transformed cell is cultured in vitro, whereby the polypeptide is obtained. The culture can be carried out according to a known method. For example, as a culture medium for an animal cell, for example, DMEM, MEM, RPMI1640, or IMDM can be used. At this time, a serum supplement such as fetal calf serum (FCS) can be used in combination or serum free culture may be carried out. The pH of the culture medium during the culture is preferably in the range of from about 6 to 8. The culture is generally carried out at about 30 to 40°C, for about 15 to 200 hours, and the culture medium may be replaced, aerated, or stirred as needed.

On the other hand, examples of the system for producing a polypeptide in vivo include a production system using an animal and a production system using a plant. A desired DNA is introduced into such an animal or a plant, and the polypeptide is produced in the animal or the plant, and then, the resulting polypeptide is recovered. The term "host" in the present invention includes such an animal and a plant. In the case where the animal is used, there is a production system using a mammal or an insect. As the mammal, goats, pigs, sheep, mice and cattle can be used (Vicki Glaser, SPECTRUM Biotechnology Applications, 1993). Further, in the case where the mammal is used, a transgenic animal can be used. For example, a desired DNA is prepared as a fusion gene with a gene encoding a polypeptide such as goat β-casein that is specifically produced in milk. Then, the resulting DNA fragment containing this fusion gene is injected into a goat's embryo, which is then implanted in a female goat. The desired polypeptide can be obtained from milk produced by a transgenic goat which is born from the goat that had received the embryo or offsprings thereof. In order to increase the amount of milk containing the polypeptide produced by the transgenic goat, a hormone may be appropriately administered to the transgenic goat (Ebert, K. M. et al., Bio/Technology (1994) 12, 699-702). Further, as the insect, for example, a silkworm can be used. In the case where the silkworm is used, by infecting the silkworm with a baculovirus into which a DNA encoding a desired polypeptide has been inserted, the desired polypeptide can be obtained from the body fluid of this silkworm (Susumu, M. et al., Nature (1985) 315, 592-594). Further, in the case where the plant is used, for example, tobacco can be used. In the case where tobacco is used, a DNA encoding a desired polypeptide is inserted into a plant expression vector such as pMON 530, and this vector is introduced into a bacterium such as Agrobacterium tumefaciens. Then, the bacterium can be used to infect tobacco such as Nicotiana tabacum, and the desired polypeptide can be obtained from the leaves of this tobacco (Julian, K. -C. Ma et al., Eur. J. Immunol. (1994) 24, 131-138). The polypeptide obtained in this way can be isolated from the inside or outside of the host cell (such as from a medium) and purified as a substantially pure homogenous polypeptide.

### Example 1

Hereinafter, Examples of the present invention will be described.

Primers for detecting A. laidlawii were produced and their detection sensitivity and specificity were confirmed. As primers for detection, the following 4 primers were produced and a primer set consisting of AF1-3 and AR1-3 was used for a primary PCR in a nested PCR and a primer set consisting of AF2-3 and AR2-3 was used for a secondary PCR. Incidentally, the primers used were chemically synthesized by a standard method.
Primer AF1-3: 5'-ttgtggtgta agtgcagt-3' SEQ ID NO: 2
Primer AF2-3: 5'-gtgcttaacg ctgtgag-3' SEQ ID NO: 3
Primer AR1-3: 5'-atctgttagc ctccgaa-3' SEQ ID NO: 4
Primer AR2-3: 5'-cctccgaact tatttctaag-3' SEQ ID NO: 5
Further, as primers for detecting other 5 species of mycoplasmas (M. hyorhinis, M. orale, M. arginini, M. fermentans and M. hominis), a primer set consisting of F1 and R1 described below was used for a primary PCR and a primer set consisting of F2 and R2 described below was used for a secondary PCR (see the Japanese Pharmacopoeia, Fourteenth Edition (2001), General Information, 9).
Primer F1: 5'-acaccatggg agytggtaat-3' SEQ ID NO: 6
Primer F2: 5'-gtgsggmtgg atcacctcct-3' SEQ ID NO: 7
Primer R1: 5'-cttcwtcgac ttycagaccc aaggcat-3' SEQ ID NO: 8
Primer R2: 5'-gcatccacca wawacyctt-3' SEQ ID NO: 9

### Confirmation of detection sensitivity

Reaction solutions with the following compositions for the primary PCR and the secondary PCR were produced and the confirmation of detection sensitivity was carried out for each of the 6 species of mycoplasmas, M. hyorhinis, M. orale, M. arginini, M. fermentans, M. hominis and A. laidlawii under the PCR reaction condition described below. The concentration of DNA sample solution of each mycoplasma was adjusted by dilution to give a final concentration of 1 pg, 100 fg and 10 fg/10 µL. DNA of each mycoplasma was prepared from about 10 mL of mycoplasma culture solution using a DNeasy Tissue Kit (manufactured by Quiagen). As for the Taq polymerase used in the PCR reaction, TaKaRa Taq HS (manufactured by TaKaRa) was used. Further, in the PCR reaction, the hot start method was used.

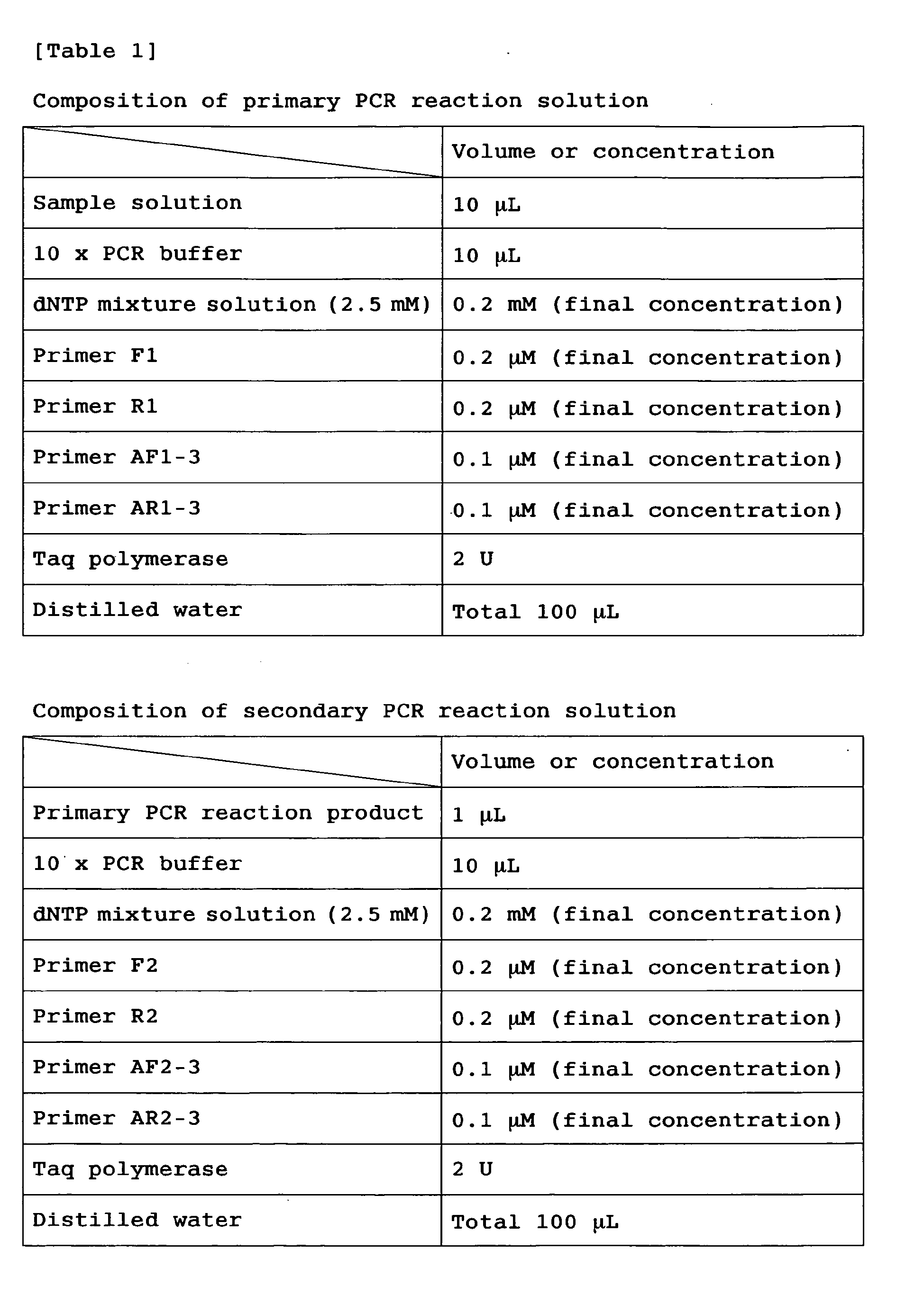

**[Table 2]**

| Primary and secondary PCR programs | | |
|---|---|---|
| Temperature | Time | Cycle number |
| 94°C | 2 min | 1 |
| 94°C | 0.5 min | 30 |
| 55°C | 2 min | |
| 72°C | 2 min | |
| 72°C | 3 min | 1 |

After the secondary PCR reaction was completed, 10 µL of the reaction solution was mixed with a 6 x loading buffer and the resulting solution was subjected to 1 x TBE agarose gel electrophoresis (1.0 %) and amplified products by the PCR were confirmed.

The results are shown in the following Table 3. As shown in the table, detection could be carried out down to 10 fg regardless of which mycoplasma DNA sample was used. Therefore, it was shown that A. laidlawii can be detected with the same sensitivity as the other 5 species of mycoplasmas. Further, because the DNA amount of 10 fg is equivalent to about several to several tens of mycoplasma cells, this detection method is considered to have a sufficient sensitivity.

**[Table 3]**

| Mycoplasma | 1 pg | 100 fg | 10 fg |
|---|---|---|---|
| M. hyorhinis | + | + | + |
| M. orale | + | + | + |
| M. arginini | + | + | + |
| M. hominis | + | + | + |
| M. fermentans | + | + | + |
| A. laidlawii | + | + | + |

### Confirmation of specificity

The specificity of the above primers for detecting A. laidlawii was confirmed by performing reactions in the same manner as the nested PCR performed in the above "Confirmation of detection specificity", except that the DNA of the sample solution was altered into the DNA (1 ng, 1 pg and 100 fg/10 µL) of 4 species of bacteria, Escherichia coli, Bacillus subtilis, Staphylococcus aureus and Pseudmonas aeruginosa. As a result, DNA amplification was not observed in any of the bacterial DNA samples. Therefore, it was confirmed that the specificity of the detection method is sufficient. Further, the specificity of the above primers for detecting A. laidlawii was confirmed by performing reactions in the same manner as described above by using the DNA (100 ng/10 µL) of 5 species of bacteria, Escherichia coli, Bacillus subtilis, Staphylococcus aureus, Pseudmonas aeruginosa and Salmonella typhimurium as the DNA of sample solutions. As a result, DNA amplification was not observed in any of the bacterial DNA samples. Therefore, it was confirmed that the specificity of this detection method is sufficient.

### (Comparative example 1)

By using a commercially available mycoplasma detection kit (ATCC Mycoplasma Detection Kit), the confirmation of detection sensitivity was carried out for each of 6 species of mycoplasmas, M. hyorhinis, M. orale, M. arginini, M. fermentans, M. hominis and A. laidlawii in the absence of sample solutions (culture solutions). The concentration of sample solutions of mycoplasma DNA was set to 1 pg, 100 fg and 10 fg/10 µL in the same manner as in Example 1. By using a 5 µL portion of the sample solution, reaction solutions with the following compositions for the primary PCR and the secondary PCR were produced and reactions were carried out with the PCR reaction conditions described below and the detection sensitivity was confirmed.

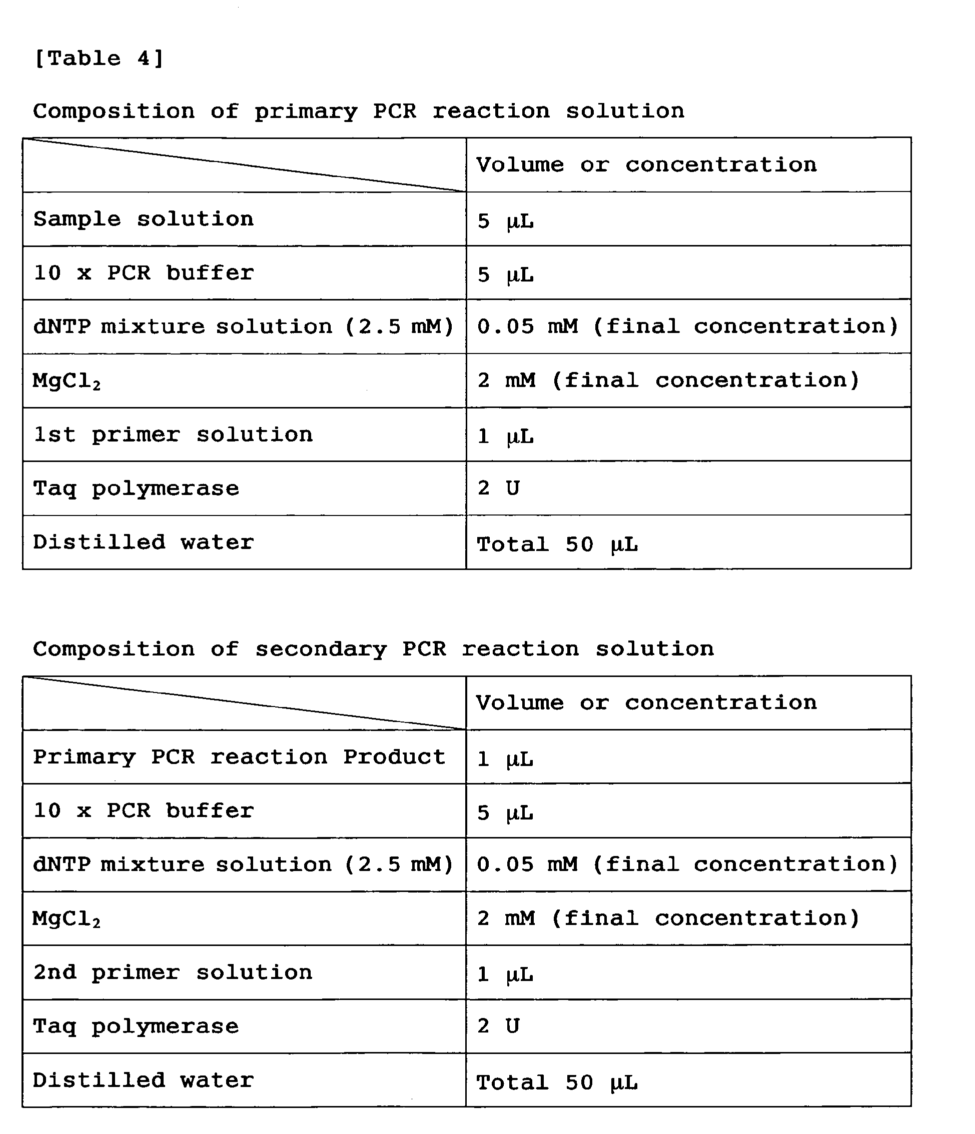

**[Table 5]**

| Primary and secondary PCR programs | | |
|---|---|---|
| Temperature | Time | Cycle number |
| 94°C | 0.5 min | 1 |
| 94°C | 0.5 min | 30 |
| 55°C | 2 min | |
| 72°C | 2 min | |
| 72°C | 5 min | 1 |

After completion of the reaction, the confirmation of DNA amplification was carried out using agarose gel electrophoresis in the same manner as in Example 1. The results are shown in the following Table 6. As shown in the table, in the absence of sample solutions, the detection sensitivity to 5 species of mycoplasmas other than A. laidlawii was 10 fg, however, the detection sensitivity to A. laidlawii was 100 fg.

**[Table 6]**

| Mycoplasma | 1 pg | 100 fg | 10 fg |
|---|---|---|---|
| M. hyorhinis | + | + | + |
| M. orale | + | + | + |
| M. arginini | + | + | + |
| M. hominis | + | + | + |
| M. fermentans | + | + | + |
| A. laidlawii | + | + | - |

### Example 2

### Confirmation of detection sensitivity in the coexistence of sample

The confirmation of detection sensitivity to mycoplasmas in the culture solution of a CSF-producing cell and in the culture solution of an anti-IL-6 receptor antibody-producing cell was carried out. The CSF culture solution is a culture solution obtained by culturing a cell described in JP-A-64-85098 and the anti-IL-6 receptor antibody culture solution is a culture solution obtained by culturing a CHO cell line that produces a humanized PM-1 antibody (an anti-human IL-6 receptor antibody) produced according to the method described in Reference example 2 in JP-A-8-99902.

To 600 µL of the CSF culture solution or the anti-IL-6 receptor antibody culture solution, 100 cfu or 10 cfu of mycoplasma was added and the cell culture solution and the mycoplasma were allowed to coexist. The mixture was centrifuged at 15000 rpm for 15 minutes and the supernatant was removed, and then, the resulting pellet was suspended in 200 µL of PBS and the suspension was used as a sample solution. Then, mycoplasma DNA was prepared from the sample solution in the same manner as in Example 1 using a DNeasy Tissue Kit and the mycoplasma in the sample solution was detected by a PCR reaction. This detection was carried out for each of the above-mentioned 6 species of mycoplasmas. The reaction conditions were as described in Tables 1 and 2.

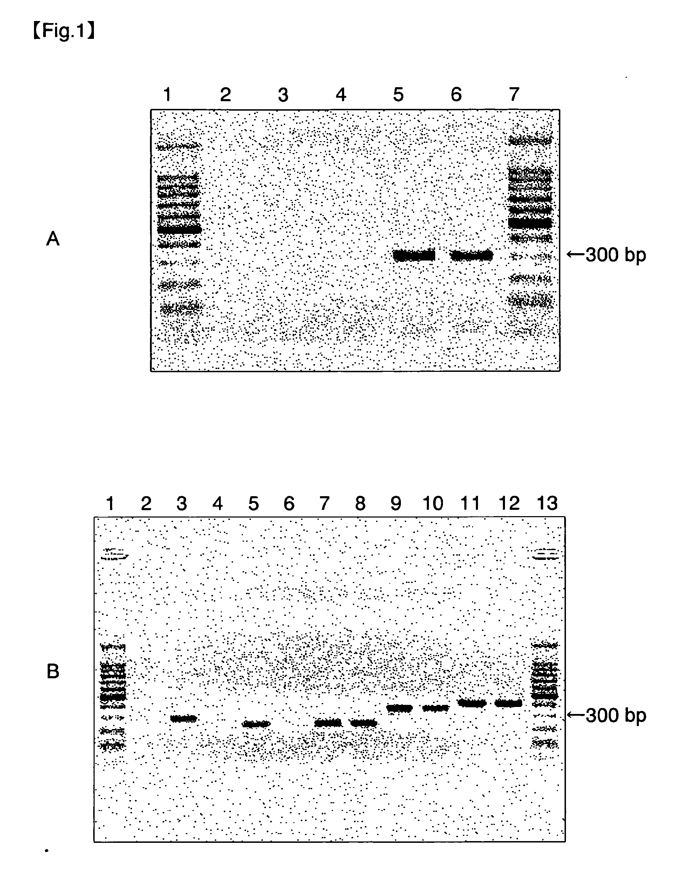

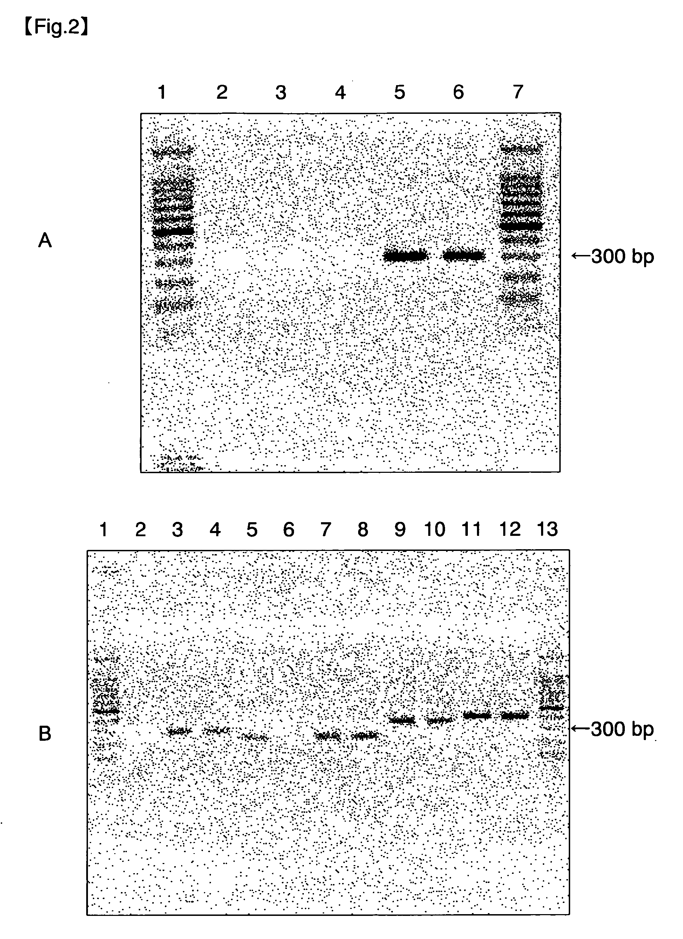

The results are shown in the following Table 7 and Figs. 1 and 2. Fig. 1A and Fig. 2A show the results of the CSF culture solution and the anti-IL-6 receptor antibody culture solution, respectively. In the drawings, the lanes 1 and 7 correspond to a molecular weight marker, the lane 2 corresponds to a negative control (distilled water), the lanes 3 and 4 correspond to the CSF culture solution and the anti-IL-6 receptor antibody culture solution, respectively, and the lanes 5 and 6 correspond to 100 cfu and 10 cfu of M. hyorhinis, respectively. Further, Fig. 1B and Fig. 2B show the results of the CSF culture solution and the anti-IL-6 receptor antibody culture solution, respectively. In the drawings, the lanes 1 and 13 correspond to a molecular weight marker, the lane 2 corresponds to a negative control (distilled water), the lanes 3 and 4 correspond to 100 cfu and 10 cfu of M. orale, respectively, the lanes 5 and 6 correspond to 100 cfu and 10 cfu of M. arginini, respectively, the lanes 7 and 8 correspond to 100 cfu and 10 cfu of M. hominis, respectively, the lanes 9 and 10 correspond to 100 cfu and 10 cfu of M. fermentans, respectively, and the lanes 11 and 12 correspond to 100 cfu and 10 cfu of A. laidlawii, respectively.

**[Table 7]**

| Mycoplasma | CSF culture solution | | Anti-IL-6R antibody culture solution | |
|---|---|---|---|---|
| | 100 cfu | 10 cfu | 100 cfu | 10 cfu |
| M. hyorhinis | + | + | + | + |
| M. orale | + | - | + | + |
| M. arginini | + | - | + | - |
| M. hominis | + | + | + | + |
| M. fermentans | + | + | + | + |
| A. laidlawii | + | + | + | + |

As shown in the above Table 7 and Figs. 1 and 2, A. laidlawii could be detected down to 10 cfu in either of the CSF culture solution and the anti-IL-6 receptor antibody culture solution.

It is known that in the case where a cultured cell is contaminated with a mycoplasma, the mycoplasma proliferates to about 10⁴ to 10⁹ cfu/mL. Therefore, it could be confirmed that contamination can be detected sufficiently with the detection sensitivity described above.

### Industrial Applicability

As described above, with the use of the primer of the present invention, a mycoplasma A. laidlawii can be detected sensitively and specifically. Further, with the use of the primer of the present invention, 6 species of mycoplasmas, M. hyorhinis, M. orale, M. arginini, M. fermentans, M. hominis and A. laidlawii, which are known to have a high infection rate of cultured cell can be detected simultaneously. Thus, the present invention is useful in a medical field and a pharmaceutical field.

### Sequence Listing Free Text

SEQ ID NO: 2: primer AF1-3
SEQ ID NO: 3: primer AF2-3
SEQ ID NO: 4: primer AR1-3
SEQ ID NO: 5: primer AR2-3
SEQ ID NO: 6: primer F1
SEQ ID NO: 7: primer F2
SEQ ID NO: 8: primer R1
SEQ ID NO: 9: primer R2

## Claims

1. A primer for amplifying at least either one of polynucleotides containing the following nucleotide sequences (A) and (B) or partial sequences thereof:
(A) a nucleotide sequence from position 589 to position 987 of SEQ ID NO: 1 or a sequence complementary thereto; and
(B) a nucleotide sequence from position 604 to position 975 of SEQ ID NO: 1 or a sequence complementary thereto.

2. The primer according to claim 1, which is for amplifying the polynucleotide containing the nucleotide sequence (A) and capable of hybridizing to at least either one of polynucleotides consisting of the following nucleotide sequences (U1) and (D1):
(U1) a sequence complementary to a nucleotide sequence from position 571 to position 588 of SEQ ID NO: 1; and
(D1) a nucleotide sequence from position 988 to position 1004 of SEQ ID NO: 1.

3. The primer according to claim 2, which is capable of hybridizing to the polynucleotide consisting of the nucleotide sequence (U1), and is at least one primer selected from the group consisting of a primer composed of a nucleotide sequence of SEQ ID NO: 2, a primer composed of a nucleotide sequence in which one or more nucleotides have been substituted, added or deleted in the nucleotide sequence of SEQ ID NO: 2, and a primer composed of a nucleotide sequence with an identity of 60% or more to the nucleotide sequence of SEQ ID NO: 2.

4. The primer according to claim 2, which is capable of hybridizing to the polynucleotide consisting of the nucleotide sequence (D1), and is at least one primer selected from the group consisting of a primer composed of a nucleotide sequence of SEQ ID NO: 4, a primer composed of a nucleotide sequence in which one or more nucleotides have been substituted, added or deleted in the nucleotide sequence of SEQ ID NO: 4, and a primer composed of a nucleotide sequence with an identity of 60% or more to the nucleotide sequence of SEQ ID NO: 4.

5. The primer according to claim 1, which is for amplifying the polynucleotide containing the nucleotide sequence (B) and capable of hybridizing to at least either one of polynucleotides consisting of the following nucleotide sequences (U2) and (D2):
(U2) a sequence complementary to a nucleotide sequence from position 587 to position 603 of SEQ ID NO: 1; and
(D2) a nucleotide sequence from position 976 to position 995 of SEQ ID NO: 1.

6. The primer according to claim 5, which is capable of hybridizing to the polynucleotide consisting of the nucleotide sequence (U2), and is at least one primer selected from the group consisting of a primer composed of a nucleotide sequence of SEQ ID NO: 3, a primer composed of a nucleotide sequence in which one or more nucleotides have been substituted, added or deleted in the nucleotide sequence of SEQ ID NO: 3, and a primer composed of a nucleotide sequence with an identity of 60% or more to the nucleotide sequence of SEQ ID NO: 3.

7. The primer according to claim 5, which is capable of hybridizing to the polynucleotide consisting of the nucleotide sequence (D2), and is at least one primer selected from the group consisting of a primer composed of a nucleotide sequence of SEQ ID NO: 5, a primer composed of a nucleotide sequence in which one or more nucleotides have been substituted, added or deleted in the nucleotide sequence of SEQ ID NO: 5, and a primer composed of a nucleotide sequence with an identity of 60% or more to the nucleotide sequence of SEQ ID NO: 5.

8. A primer set, which is for detecting a mycoplasma A. laidlawii and includes 2 or more types of primers, comprising at least 2 types of primers according to claim 1.

9. The primer set according to claim 8, which is to be used in a nested PCR method, including the following primers (i) and (ii) as primers for a primary PCR, and the following primers (iii) and (iv) as primers for a secondary PCR:
(i) a primer capable of hybridizing to a polynucleotide containing a sequence complementary to a nucleotide sequence from position 571 to position 588 of SEQ ID NO: 1;
(ii) a primer capable of hybridizing to a polynucleotide containing a nucleotide sequence from position 988 to position 1004 of SEQ ID NO: 1;
(iii) a primer capable of hybridizing to a polynucleotide containing a sequence complementary to a nucleotide sequence from position 587 to position 603 of SEQ ID NO: 1; and
(iv) a primer capable of hybridizing to a polynucleotide containing a nucleotide sequence from position 976 to position 995 of SEQ ID NO: 1.

10. A mycoplasma detection kit, which is for detecting a mycoplasma by gene amplification, comprising the primer according to claim 1 as a primer to be used for the gene amplification for detecting a mycoplasma A. laidlawii.

11. The mycoplasma detection kit according to claim 10, further comprising one or more primers to be used for the gene amplification for detecting at least one mycoplasma selected from the group consisting of Mycoplasma hyorhinis, Mycoplasma orale, Mycoplasma arginini, Mycoplasma fermentans and Mycoplasma hominis.

12. The mycoplasma detection kit according to claim 11, including at least one primer selected from the group consisting of a primer composed of a nucleotide sequence of SEQ ID NO: 6, a primer composed of a nucleotide sequence of SEQ ID NO: 7, a primer composed of a nucleotide sequence of SEQ ID NO: 8, and a primer composed of a nucleotide sequence of SEQ ID NO: 9 as the primer to be used for the gene amplification for detecting at least one mycoplasma selected from the group consisting of Mycoplasma hyorhinis, Mycoplasma orale, Mycoplasma arginini, Mycoplasma fermentans and Mycoplasma hominis.

13. A detection method for detecting a mycoplasma Acholeplasma laidlawii, comprising amplifying at least either one of polynucleotides containing the following nucleotide sequences (A) and (B) or partial sequences thereof and detecting the amplified polynucleotide:
(A) a nucleotide sequence from position 589 to position 987 of SEQ ID NO: 1 or a sequence complementary thereto; and
(B) a nucleotide sequence from position 604 to position 975 of SEQ ID NO: 1 or a sequence complementary thereto.

14. The detection method according to claim 13, wherein the primer according to claim 1 is used in the amplification of the polynucleotide.

15. A method for producing a pharmaceutical composition, comprising the step of examining a mycoplasma in a produced pharmaceutical composition, wherein the examination step is carried out by the detection method according to claim 13 or 14.

16. A method for producing a pharmaceutical composition comprising a polypeptide, the method comprising the steps of:
(i) culturing a host cell containing a gene encoding the polypeptide contained in the pharmaceutical composition;
(ii) collecting a polypeptide expressed in the host cell in (i);
(iii) obtaining a sample for examination from the polypeptide collected in (ii); and
(iv) examining that a mycoplasma is not present in the sample obtained in (iii) by the detection method according to claim 13 or 14.

17. A method for producing a pharmaceutical composition containing a polypeptide, the method comprising the steps of:
(i) culturing a host-cell containing a gene encoding the polypeptide contained in the pharmaceutical composition;
(ii) collecting a polypeptide expressed in the host cell in (i);
(iii) obtaining a sample for examination from the polypeptide collected in (ii); and
(iv) examining that a mycoplasma is not present in the sample obtained in (iii) by using the primer according to any one of claims 1 to 7, the primer set according to claim 8 or 9, or the detection kit according to any one of claims 10 to 12.
